(19) Europäisches Patentamt / European Patent Office / Office européen des brevets

(11) **EP 1 923 056 B1**

(12) **EUROPÄISCHE PATENTSCHRIFT**

(45) Veröffentlichungstag und Bekanntmachung des Hinweises auf die Patenterteilung:
**19.10.2011 Patentblatt 2011/42**

(51) Int Cl.:
*A61K 9/20* (2006.01)     *A61K 31/138* (2006.01)
*A61K 31/485* (2006.01)

(21) Anmeldenummer: **07120927.4**

(22) Anmeldetag: **16.11.2007**

(54) **Pharmazeutische Einzeldosisform**

Pharmaceutical single dosage form

Forme galénique individuelle

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **16.11.2006 DE 102006054638**

(43) Veröffentlichungstag der Anmeldung:
**21.05.2008 Patentblatt 2008/21**

(73) Patentinhaber: **Laburnum GMBH**
**16540 Hohen Neuendorf (DE)**

(72) Erfinder: **Ronca, Renzo**
**16540, Hohen Neuendorf (DE)**

(74) Vertreter: **Schubert, Klemens**
**Neue Promenade 5**
**10178 Berlin-Mitte (DE)**

(56) Entgegenhaltungen:

| | |
|---|---|
| EP-A- 1 306 071 | WO-A-2006/000229 |
| DE-A1- 2 922 522 | US-A- 4 322 449 |
| US-A- 4 754 597 | US-A- 4 985 252 |
| US-A1- 2004 048 831 | US-B1- 6 399 591 |

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 923 056 B1

**Beschreibung**

Die vorliegende Erfindung betrifft pharmazeutische Einzeldosisformen Hers telly derselben sowie ein Kit zur

Es besteht ein großer Bedarf an exakt dosierten Einzeldosisformen. Derartige Einzeldosisformen sind beispielsweise für Kinder erforderlich, da diese wegen des geringeren Körpergewichts oft die für Erwachsene vorgesehene Wirkstoffdosierung nicht vertragen. In solchen Fällen wird dann eine herkömmliche Tablette mit Bruchrillen versehen, um verschiedene Dosierungsstärken zu erhalten. Die Teilung solcher Tabletten ist aber mit vielen Nachteilen behaftet. So brechen diese Tabletten oft nicht exakt genug, so dass es dennoch zu Unter- oder gar Überdosierungen kommt. Dies führt dann dazu, dass für den Wirkstoff nicht die erforderlichen Wirkstoffspiegel im Blut erreicht werden.

Andere Dosierungsformen, wie beispielsweise Tropfen, können zwar individueller dosiert werden, aber hier besteht die Gefahr, dass sich der Patient beim Zählen der Tropfen irrt und somit ebenfalls eine falsche Dosierung eintritt.

Ein weiteres Problem besteht bei der Herstellung von individuellen, vom Arzt verordneten Zubereitungen. Diese werden regelmäßig in Apotheken hergestellt und stellen sehr hohe Anforderungen an Vorrichtungen und Personal. Der Wirkstoff muss in eine für die Applikation geeignete Zubereitung gebracht werden, welche die Anforderungen an die Dosierungsgenauigkeit erfüllen, um keine Fehldosierungen zu bewirken.

Bei all diesen Einzelformen ist es nicht ohne weiteres möglich, in einfacher Weise exakte und individuelle Dosierungen zu erzielen. Auch können keine einzeln zubereitete Arzneimittel auf diese Weise hergestellt werden.

EP-A-1 306 071 offenbart einen blattförmigen, essbaren Träger, auf welchen tropfenweise bioaktive Flüssigkeiten aufgebracht werden (ähnlich einem Tintenstrahldrucker). Die Herstellung fester Arzneiformen wie Tabletten oder Kapseln wird nicht offenbart. EP-A-1 306 071 beschreibt auch keine Einzeldosisform, da der blattförmige Träger durch Stanzen oder Schneiden zunächst in Einzeldosisformen zerteilt werden müsste.

US-A-4 754 597 offenbart feste, geformte Artikel, bei denen es sich insbesondere um schnell lösliche Dosierformen handelt. Erzeugt wird ein Netzwerk von Trägermaterial, auf das eine Lösung oder Suspension eines nichtwässrigen Lösemittels aufgebracht wird, das eine chemische Verbindung enthält. Abschließend wird das Lösemittel abgedampft. Hierbei wird bevorzugt das Gefriertrocknungsverfahren verwendet.

US 2004/048831 A1 beschreibt feste Dosierformen, die Cyclodextrin umfassen. Ein biologisch aktiver Stoff, der befähigt sein muss, mit Cyclodextrin eine Einschlussverbindung zu bilden, wird in ein wässriges Lösemittel eingebracht. Hinzugefügt wird Cyclodextrin, wodurch sich eine Einschlussverbindung des Wirkstoffs in Cyclodextrin bildet, welche auf eine Trägermatrix aufgebracht wird.

WO 2006/000229 A beschreibt mit Wirkstoff beladene Tabletten und deren Herstellung. Erfindungsgemäß wird der Wirkstoff auf einen Rohling gesprüht oder der Rohling wird in eine wirkstoffhaltige Lösung eingebracht, damit er diese aufnehmen kann.

DE 25 22 522 A1 beschreibt eine Tablette mit einem Trägermaterial, das einen Wirkstoff enthält. Das Trägermaterial ist als saugfähiges Fasermaterial ausgebildet. Der Wirkstoff wird auf der Oberfläche des Trägermaterials aufgebracht oder in dieses eingebettet.

US-B1-6 399 591 offenbart eine wirkstoffhaltige Tablette, die hergestellt wird, indem ein aktiver Bestandteil in flüssiger Form auf die ausgeformte Tablette aufgebracht wird. Gegebenenfalls wird die mit Wirkstoff beladene Tablette zentrifugiert, um Lösemittel zu entfernen.

US-A-4 985 252 beschreibt pulverförmige Zusammensetzungen. Auf einer äußeren oder inneren Oberfläche von Partikeln ist ein aktiver Bestandteil adsorbiert. Hergestellt werden, diese Pulver, indem ein aktiver Bestandteil in einem Lösemittel gelöst wird, diese Lösung mit einem Zucker versetzt und anschließend das Lösemittel abgedampft wird.

US-A-4 322 449 offenbart U. a. die Herstellung von Tabletten, wobei auf ein Trägermaterial tropfenförmig Wirkstoff in einer Flüssigkeit aufgebracht wird. Anschließend wird das mit dem Wirkstoff versetzte Trägermaterial zu einer Tablette verpresst.

Aufgabe der vorliegenden Erfindung ist es daher, pharmazeutische Einzeldosisformen zur Verfügung zu stellen, welche die Nachteile des Standes der Technik überwinden.

Die Aufgabe wird durch eine pharmazeutische Einzeldosisform gemäß dem Hauptanspruch gelöst.

Erfindungsgemäß ist dabei, dass das Aufbringen mittels einer Dosierpumpe und/oder einer Pipette erfolgt. Geeignete Dosierpumpen zum Aufbringen der wirkstoffhaltige Lösung sind beispielsweise Hubkolben-, Schlauch-, Membran- oder Zahnradpumpen. Ferner haben sich Pipetten, insbesondere Kolbenhubpipetten mit aufgesetzten Kunststoffpipettenspitzen, beispielsweise Eppendorf® Multipetten® mit Combitips®, zum Aufbringen der wirkstoffhaltigen Lösung als besonders geeignet erwiesen, da mit ihnen ein einfaches und genaues Dosieren möglich ist.

Das Lösungsmittel dient zum Aufbringen des Wirkstoffes auf den Träger und wird anschließend entfernt. Dies kann durch dem Fachmann bekannte Verfahren, wie beispielsweise Abziehen im Vakuum, geschehen.

Es ist daher erfindungsgemäß bevorzugt, dass die wirkstoffhaltige Lösung ein pharmazeutisch unbedenkliches Lösungsmittel enthält. Als pharmazeutisch unbedenkliche Lösungsmittel können beispielsweise Wasser, Aceton, Alkohole, insbesondere Methanol, Ethanol, Propanol oder Isopropanol, Glykole, insbesondere Popylenglykol, MCT (medium chain triglycerides [Triglyceride von Fettsäuren mittlerer Kettenlänge]) und/oder Erdnussöl verwendet werden. Auch die Verwendung von geeigneten Lösungsmittelgemischen ist erfindungsgemäß vorgesehen.

Der Träger besteht vorzugsweise aus pharmazeutisch verträglichen Trägerstoffen und/oder pharmazeutisch verträglichen Hilfsstoffen. Durch die Verwendung entsprechend geeigneter, dem Fachmann bekannter Hilfs- und Trägerstoffe lassen sich die pharmakokinetischen und pharmakodynamischen Eigenschaften des erfindungsgemäßen Arzneimittels einstellen. Hierbei können je nach den gewünschten Eigenschaften des herzustellenden Trägers Füll-, Binde-, Spreng-, Gleit- und Schmiermittel, Mittel, die das Verhalten der Träger im Verdauungsapparat verändern können, zugelassene Farbstoffe und Geschmackskorrigentien zur Herstellung des Trägers verwendet werden.

In einer bevorzugten Ausführungsform der Erfindung liegen zwei oder mehrere Träger miteinander zu einem Träger kombiniert vor, wobei auf den Trägern unterschiedliche pharmazeutische Wirkstoffe aufgetragen sind oder die Träger aus unterschiedlichen pharmazeutischen Trägerstoffen und/oder Hilfsstoffen bestehen.

Die erfindungsgemäße Einzeldosisform kann somit aus einem Träger bestehen, der aus mehreren Trägerschichten mit unterschiedlichen Wirkstoffen oder unterschiedlichen Hilfs- und/oder Trägerstoffen besteht.

Ein weiterer Gegenstand der vorliegenden Erfindung ist ein Träger für erfindungsgemäße pharmazeutische Einzeldosisformen, welcher pharmazeutisch verträgliche Trägerstoffe und/oder pharmazeutisch verträgliche Hilfsstoffe enthält. Es ist jedoch auch denkbar, dass der Träger selbst teilweise oder vollständig aus einem Wirkstoff besteht.

Erfindungsgemäß ist es dabei, dass der Träger in Form eines Presslings vorliegt. Die Presslinge können erfindungsgemäß so ausgebildet sein, dass diese nach dem Aufbringen des Wirkstoffes in eine Hartgelatinekapsel gegeben werden können, die dann verschlossen wird.

Weiterhin istes erfindungsgemäß, dass der Träger wenigstens an einer Oberfläche Vertiefungen und/oder Erhöhungen aufweist, welche die Oberfläche vergrößern. So weist die Oberfläche des Trägers eine oder mehrere Mulden auf. Durch diese Mulde und/oder eine poröse Oberfläche wird das Aufbringen des gelösten Wirkstoffes erleichtert. Zudem wird durch eine größere Oberfläche die Aufnahmegeschwindigkeit erhöht.

Die Eindringtiefe der Lösungsmittel, sowie die Handhabbarkeit beziehungsweise die Verwendbarkeit der Träger wird durch Wärme, Infrarotstrahlung oder Vakuum gesteuert.

Träger, die die Aufnahme von Wirkstofflösungen auf der Basis von Ethanol (100 %), Ethanol (96 %), Aceton, Isopropanol, Propylenglykol, MCT und Erdnussöl sowie Mischungen dieser untereinander, nebst Zusätzen von Wasser unter gegebenenfalls Zuhilfenahme von Lösungsvermittlern wie Polyvinylpyrrolidon (PVP), Polysorbat 80 und/oder Ammoniak sind entwickelt worden.

Weitere Varianten des Trägers sind beispielsweise:

a) Zwei- oder Mehrschichttabletten
Zwei- oder Mehrschichttabletten können unterschiedliche Wirkstoffe in den einzelnen Schichten enthalten oder aus Hilfsstoffkombinationen bestehen, durch die die einzelnen Schichten unterschiedliche Wirkstofffreisetzungseigenschaften besitzen.

b) vorgefertigte "konventionelle" wirkstoffhaltige Tabletten mit anschließender Wirkstoffbeigabe durch das erfindungsgemäße Verfahren

c) Mischformen aus (a) und (b)

Physische Parameter können erfindungsgemäß beispielsweise das Gewicht, die Körpergröße und/oder die Körperoberfläche oder Kombinationen daraus sein. Aber auch andere besondere Eigenschaften wie beispielsweise die Schmerzempfindlichkeit.

Für eine medikamentöse Therapie ist eine adäquate Dosierung, d.h. eine Dosierung, bei der der gewünschte Effekt ohne Überdosierung und daher ohne vermeidbare toxische Nebenwirkungen erreicht wird, zu beachten. Der vom Arzneimittel im Körper ausgelöste Effekt ist von der Konzentration am Wirkort und damit, bei einer konstanten Dosis, von den physischen Parametern, wie beispielsweise vom Körpergewicht des Patienten, abhängig. Zur Vereinfachung der Dosierung wird bei Erwachsenen von einem Durchschnittsgewicht von 70 kg ausgegangen. Da die Pharmakokinetik bei Kindern von der eines Erwachsenen abweicht, muss die zu applizierende Dosis dementsprechend reduziert werden.

Die Kinderdosis kann vielfach unter Berücksichtigung der Körperoberfläche des Kinds errechnet werden.

Die Normdosis (ND) für ein Kind kann beispielsweise durch die folgende Formel berechnet werden:

$$ND_{Kind} = ND_{Erwachsener} \cdot \frac{KO}{1,73}$$

Mit KO als Körperoberfläche in m$^2$. Die Körperoberfläche kann angenähert durch folgende Formel erhalten werden:

$$KO = 0{,}09 \cdot W^{0{,}73}$$

Mit W als Körpergewicht in kg.

Durch das erfindungsgemäße einzeln zubereitete Arzneimittel wird eine Einzeldosisform bereitgestellt, die es ermöglicht, einen pharmazeutischen Wirkstoff individuell für einen Patienten herzustellen. Die benötigte Wirkstoffmenge kann auf Basis einer Rezeptur eines Arztes oder, wie oben für die Dosierung bei Kindern dargestellt, errechnet werden.

Ein solches erfindungsgemäß einzeln zubereitetes Arzneimittel kann sowohl im humanmedizinischen als auch im veterinärmedizinischen Bereich eingesetzt werden.

Die pro Individuum benötigte Menge an pharmazeutischem Wirkstoff ist vorzugsweise beim Aufbringen auf den Träger in einem definierten Volumen eines Lösungsmittels gelöst. Zur Hilfestellung für den Arzt oder Apotheker, der das erfindungsgemäße Arzneimittel herstellt, können Tabellen bereitgestellt werden, anhand derer man das benötigte Volumen der wirkstoffhaltigen Lösung ablesen kann, um so eine genau definierte Menge der wirkstoffhaltigen Lösung auf den Träger aufzubringen.

Die Applikation des Wirkstoffes kann mit dem erfindungsgemäßen Verfahren genau auf die physischen Parameter eines Individuums abgestimmt werden. Zur Berechnung der Applikationsmenge dienen die in der Tabelle 1 angegebenen Werte.

Tabelle 1:

| Nennvolumen | Konzentration der Lösung in % | | | | | | | | | | | | | | | übertragene Masse |
|---|---|---|---|---|---|---|---|---|---|---|---|---|---|---|---|---|
| µl | 0,01 | 0,015 | 0,05 | 0,1 | 0,5 | 1 | 2 | 3 | 4 | 5 | 6 | 7 | 8 | 9 | 100 | |
| 10 | 1 | 1,5 | 5 | 10 | 50 | 100 | 200 | 300 | 400 | 500 | 600 | 700 | 800 | 900 | 1000 | in µg |
| 20 | 2 | 3 | 10 | 20 | 100 | 200 | 400 | 600 | 800 | 1000 | 1200 | 1400 | 1600 | 1800 | 2000 | in µg |
| 30 | 3 | 4,5 | 15 | 30 | 150 | 300 | 600 | 900 | 1200 | 1500 | 1800 | 2100 | 2400 | 2700 | 3000 | in µg |
| 40 | 4 | 6 | 20 | 40 | 200 | 400 | 800 | 1200 | 1600 | 2000 | 2400 | 2800 | 3200 | 3600 | 4000 | in µg |
| 50 | 5 | 7,5 | 25 | 50 | 250 | 500 | 1000 | 1500 | 2000 | 2500 | 3000 | 3500 | 4000 | 4500 | 5000 | in µg |
| 60 | 6 | 9 | 30 | 60 | 300 | 600 | 1200 | 1800 | 2400 | 3000 | 3600 | 4200 | 4800 | 5400 | 6000 | in µg |
| 70 | 7 | 10,5 | 35 | 70 | 350 | 700 | 1400 | 2100 | 2800 | 3500 | 4200 | 4900 | 5600 | 6300 | 7000 | in µg |
| 80 | 8 | 12 | 40 | 80 | 400 | 800 | 1600 | 2400 | 3200 | 4000 | 4800 | 5600 | 6400 | 7200 | 8000 | in µg |
| 90 | 9 | 13,5 | 45 | 90 | 450 | 900 | 1800 | 2700 | 3600 | 4500 | 5400 | 6300 | 7200 | 8100 | 9000 | in µg |
| 100 | 10 | 15 | 50 | 100 | 500 | 1000 | 2000 | 3000 | 4000 | 5000 | 6000 | 7000 | 8000 | 9000 | 10000 | in µg |

Gemäß der in der Tabelle 1 dargestellten Werte kann, für die Herstellung einer Tablette mit 5 mg Wirkstoff, eine wirkstoffhaltige Lösung mit 5 % Gehalt eins Wirkstoffes genutzt werden, um ein Volumen von insgesamt 100 $\mu$l auf den wirkstofflosen Träger aufzubringen.

Der zu applizierende Wirkstoff in wird in einer definierten Konzentration gelöst. Anschließend wird dann mit der Dosierpumpe und/oder Pipette ein definiertes Volumen der wirkstoffhaltigen Lösung auf den Träger aufgebracht.

Erfindungsgemäß bevorzugt ist dabei auch, dass man die so erhaltene Einzeldosis mit einem Überzug versieht oder in eine Gelatinekapsel füllt.

Wie oben beschrieben können die Träger in weiteren Verfahrensschritten zu Schichttabletten weiterverarbeitet werden.

Weiterhin wird die Aufgabe der vorliegenden Erfindung durch ein Kit gelöst, mit den Merkmaten des Anspruchs 5, Hierin kann der Wirkstoff getrennt vom Lösungsmittel oder bereits in einer definierten Konzentration als Lösung vorliegen.

Die erfindungsgemäßen pharmazeutischen Einzeldosisformen weisen gegenüber dem Stand der Technik eine Reihe von Vorteilen auf. Sie gestatten eine exakte und individuelle Dosierung von Wirkstoffen. Dies erlaubt die Herstellung von Arzneimitteln für Kinder, da eine individuelle Dosierung nach Alter und Gewicht einfach bewerkstelligt werden kann. Entsprechendes gilt auch bei Patienten mit Niereninsuffizienz, da diese auf individuell dosierte Arzneimittel angewiesen sind, um die Nierenfunktion nicht weiter zu schädigen.

Die erfindungsgemäßen pharmazeutischen Einzeldosisformen erlauben eine einfache Herstellung von Kleinserien und Einzelanfertigungen.

Die erfindungsgemäßen pharmazeutischen Einzeldosisformen können somit insbesondere auch in der klinischen Forschung als Prüfmuster verwendet werden, um insbesondere Erstanwendungen durchzuführen.

Die erfindungsgemäßen Träger weisen gleichfalls eine Reihe von Vorteilen auf. Sie können in an sich bekannter Weise aus den üblichen pharmazeutischen Trägerstoffen sowie Hilfsstoffen hergestellt werden. Da in diesen Formen der Wirkstoff nicht eingearbeitet werden muss, lassen sich diese Träger unabhängig vom jeweiligen Wirkstoff zubereiten. Dabei lassen sich auch laktosefreie Träger herstellen sowie Träger, in denen bestimmte Hilfsstoffe nicht enthalten sind. Dadurch können beispielsweise auch bestimmte Diäten unterstützt und Unverträglichkeiten für bestimmte Stoffe beachtet werden.

Da die Träger für alle Einzeldosisformen gleich sein können, ist eine kostengünstige und leicht validierbare Herstellung der wirkstoffhaltigen Formen gegeben. Dies führt zu äußerst preiswerten, einzeldosierten Arzneimitteln. Dies bedeutet, dass der Arzt individuelle Verordnungen vorgeben kann, welche dann sehr preisgünstig herstellbar sind.

Die Träger können dann, nachdem der Wirkstoff aufgebracht wurde, mit Überzügen versehen werden, um geschmackliche Korrekturen anzubringen. Mit derartigen Überzügen entstehen dann an sich bekannte Formen, wie Filmtabletten, Dragees, magensaftresistente Tabletten bzw. Dragees und dergleichen.

Die mit Wirkstoff versehenen Träger können aber auch in Gelatinekapseln eingebracht werden.

Die Herstellung der Träger ist an sich bekannt. Dazu werden geeignete pharmazeutisch verträgliche Trägerstoffe und/oder pharmazeutisch verträgliche Hilfsstoffe gemischt und verpresst. Die entsprechenden Techniken sind beispielsweise beschrieben in "Hagers Handbuch der pharmazeutischen Praxis Band 2 Methoden", Hrsg. E, Nürnberg, P. Surmann, 5. Auflage, 1991, Springer-Verlag. Die Vertiefungen auf der Oberseite und/oder Unterseite werden durch entsprechend geformte Stempel und Matrizen erzeugt. Auch dies ist dem Fachmann bekannt.

Im Folgenden wird die Erfindung anhand von Beispielen näher beschrieben.

<u>Träger:</u>

Als Träger wurden die folgenden Substanzen in unterschiedlichen Mengen und divergierenden Mischungen verwendet:

Mannitol, Sorbitol, Alginsäure, $\beta$-Cyclodextrin, Zeolithe, Polyvinylacetate/Polyvinylpyrrolidone (8+2), vorgekleisterte Maisstärke, mikrokristalline Cellulose, Talcum, Magnesiumstearat, u.a.

Durch die Kombination dieser Bestandteile ist eine Wirkstoffabgabe in unterschiedlichen Zeitintervallen möglich, dies erlaubt die Herstellung von Retardpräparaten.

<u>Wirkstoffdosierung am Beispiel Propranolol:</u>

Als Anfangsdosis werden 70 mg pro m$^2$ Körperoberfläche gegeben. Die Anfangsdosis im Alter von 3 Monaten liegt bei 3,6 mg und bei Erwachsenen 1,8 mg pro kg Körpergewicht.

Die Dosierung (Tagesdosis) von Propranolol ist in Tabelle 2 dargestellt.

Tabelle 2:

| Alter (in Jahren) | durchschnittliches Körpergewicht (in kg) | Dosiseinheit (peroral) (in mg) |
|---|---|---|
| ¼ | 5,5 | 20 |
| ½ | 7,5 | 25 |
| 1 | 10 | 30 |
| 3 | 14 | 40 |
| 7½ | 24 | 60 |
| 12 | 38 | 80 |
| Erwachsener | 65 | 120 |

Beispiel einer Einzeldosisform:

wässrig (rapid) Morphinsulfat

Dextrose, gehärtetes pflanzliches Öl (beispielsweise Rapsöl, Cocosölderivate) > 2 %, Magnesiumstearat q.s. (quantum satis) ad 2500 mg
Die Haltbarkeit dieser Trägerzubereitung liegt bei über 30 Tagen. Eine Maillard-Reaktion wurde nicht beobachtet.

Löslichkeit von Morphinsulfat:

In den Tabellen 3 und 4 ist die Löslichkeit von Morphinsulfat (Pentahydrat) bei 25 °C (Tabelle 3) und bei 80 °C (Tabelle 4) dargestellt.

Tabelle 3:

| Morphinsulfat (Pentahydrat) | Lösungsmittel (Wasser) |
|---|---|
| 10 mg | 0,16 ml |
| 20 mg | 0,31 ml |
| 30 mg | 0,47 ml |
| 50 mg | 0,78 ml |
| 100 mg | 1,55 ml |
| 250 mg | 3,88 ml |
| 500 mg | 7,75 ml |
| 1000 mg | 15,5 ml |

Tabelle 4:

| Morphinsulfat (Pentahydrat) | Lösungsmittel (Wasser) |
|---|---|
| 30 mg | 0,02 ml |
| 50 mg | 0,04 ml |
| 100 mg | 0,07 ml |
| 250 mg | 0,18 ml |
| 500 mg | 0,35 ml |
| 1000 mg | 0,7 ml |

Die zu applizierende Einzeldosis an Morphinsulfat ist vom Individuum abhängig und hängt von dessen Alter, Größe und Körpergewicht sowie dessen Schmerzempfinden ab. Als Richtwert geht man von einer Einzeldosis (peroral) von 5 mg für 3-5 jährige, von 5-10 mg für 6-12 jährige und von 20 mg für Erwachsene aus.

## Patentansprüche

1. Pharmazeutische Einzeldosisform für ein bestimmtes Individuum in Form von Tabletten, erhältlich durch Aufbringen einer wirkstoffhaltigen Lösung auf einen festen Träger, in form eines. Presslings, wobei die Menge des aufgebrachten Wirkstoffes anhand physischer Parameter des Individuums bestimmt ist, für welches das Arzneimittel vorgesehen ist, **dadurch gekennzeichnet, dass** der Träger an einer Oberfläche eine oder mehrere Mulden aufweist, in welche die wirkstoffhaltige Lösung aufgebracht wird, und wobei die Eindringtiefe der Lösungsmittel durch Wärme, Infrarotstrahlung oder Vakuum gesteuert wird, und**dass** das Aufbringen mittels einer Dosierpumpe und/oder einer Pipette erfolgt.

2. Pharmazeutische Einzeldosisform gemäß Anspruch 1, **dadurch gekennzeichnet, dass** die wirkstoffhaltige Lösung ein pharmazeutisch unbedenkliches Lösungsmittel enthält.

3. Pharmazeutische Einzeldosisform gemäß einem der Ansprüche 1 bis 2, **dadurch gekennzeichnet, dass** der Träger pharmazeutisch verträgliche Trägerstoffe und/oder pharmazeutisch verträgliche Hilfsstoffe enthält.

4. Pharmazeutische Einzeldosisform gemäß einem der Ansprüche 1 bis 3, **dadurch gekennzeichnet, dass** zwei oder mehrere Träger miteinander zu einem Träger kombiniert vorliegen,
wobei auf den Trägern unterschiedliche pharmazeutische Wirkstoffe aufgetragen sind oder
die Träger aus unterschiedlichen pharmazeutisch verträglichen Trägerstoffen und/oder Hilfsstoffen bestehen.

5. Kit zur Herstellung einer pharmazeutisohen Einzeldosis form gemäß einem der Ansprüche, 7 bis 4, einen Träger, ein Lösungsmittel und einen pharmazeutischen Wirkstoff und eine Dosierpumpe und/oder eine Pipette enthaltend.

## Claims

1. Pharmaceutical single dose unit for a certain individual in the form of tablets, obtainable by applying a solution containing an active ingredient onto a solid carrier, in the form of a compressed core, wherein the amount of the applied active ingredient is defined on the basis of physical parameters of the individual, the medicament is intended for,
**characterized in that** the carrier has one ore more depressions at one surface thereof, wherein the solution containing the active ingredient is applied, and wherein the penetration depth of the solvents is regulated by heat, infrared radiation or vacuum, and **in that** the application is carried out by use of a dosing pump and/or a pipette.

2. Pharmaceutical single dose unit according to claim 1, **characterized in that** the solution containing the active ingredient contains a pharmaceutical compatible solvent.

3. Pharmaceutical single dose unit according to any of claims 1 to 2, **characterized in that** the carrier contains pharmaceutical acceptable additives and carrier substances.

4. Pharmaceutical single dose unit according to any of claims 1 to 3, **characterized in that** two or more carriers are combined to make up one carrier, wherein different pharmaceutical active ingredients are applied on the carriers, or **in that** the carriers consist of different pharmaceutical carrier substances and/or additives.

5. Kit for the preparation of a pharmaceutical single dose unit according to any of claims 1 to 4, consisting of a carrier according to claim 1, a solvent and a pharmaceutical ingredient and a dosing pump and/or a pipette.

## Revendications

1. Forme de dose individuelle pharmaceutique pour un individu déterminé sous forme de comprimés, pouvant être obtenue par application d'une solution contenant une substance active sur un support solide, sous forme d'une

EP 1 923 056 B1

pièce pressée, la quantité de substance active appliquée étant déterminée à l'aide de paramètres physiques de l'individu, pour lequel le médicament est prévu, **caractérisée en ce que** le support présente sur une surface un ou plusieurs creux dans lequel/lesquels la solution contenant la substance active est appliquée et où la profondeur de pénétration des solvants est contrôlée par de la chaleur, un rayonnement infrarouge ou un vide et **en ce que** l'application est réalisée au moyen d'une pompe de dosage et/ou d'une pipette.

2. Forme de dose individuelle pharmaceutique selon la revendication 1, **caractérisée en ce que** la solution contenant la substance active contient un solvant pharmaceutiquement inoffensif.

3. Forme de dose individuelle pharmaceutique selon l'une quelconque des revendications 1 à 2, **caractérisée en ce que** le support contient des substances support pharmaceutiquement acceptables et/ou des adjuvants pharmaceutiquement acceptables.

4. Forme de dose individuelle pharmaceutique selon l'une quelconque des revendications 1 à 3, **caractérisée en ce que** deux supports ou plus se trouvent sous une forme combinée les uns avec les autres en formant un support, des substances actives pharmaceutiques différentes étant appliquées sur les supports ou les supports étant constitués par des substances support et/ou des adjuvants pharmaceutiquement acceptables différent(e)s.

5. Kit pour la préparation d'une forme de dose individuelle pharmaceutique selon l'une quelconque des revendications 1 à 4, contenant un support selon la revendication 1, un solvant et une substance active pharmaceutique et une pompe de dosage et/ou une pipette.

## IN DER BESCHREIBUNG AUFGEFÜHRTE DOKUMENTE

### In der Beschreibung aufgeführte Patentdokumente

- EP 1306071 A
- US 4754597 A
- US 2004048831 A1
- WO 2006000229 A
- DE 2522522 A1
- US 6399591 B1
- US 4985252 A
- US 4322449 A

### In der Beschreibung aufgeführte Nicht-Patentliteratur

- Hagers Handbuch der pharmazeutischen Praxis Band 2 Methoden. Springer-Verlag, 1991, vol. 2